**Europäisches Patentamt**

**European Patent Office**

**Office européen des brevets**

(11) Veröffentlichungsnummer: **0 240 735**

**A2**

# (12) EUROPÄISCHE PATENTANMELDUNG

(21) Anmeldenummer: 87103210.8

(22) Anmeldetag: 06.03.87

(51) Int. Cl.³: **A 61 B 5/02**

(30) Priorität: 24.03.86 DE 3609912

(43) Veröffentlichungstag der Anmeldung:
14.10.87 Patentblatt 87/42

(84) Benannte Vertragsstaaten:
AT BE CH DE ES FR GB GR IT LI LU NL SE

(71) Anmelder: Ernst, Franz T., Dr. med.
Haubachstrasse 6
D-4600 Dortmund 50(DE)

(71) Anmelder: Jünemann, Reinhard, Prof. Dr.-Ing.
Goerdeler Strasse 24
D-4600 Dortmund 50(DE)

(72) Erfinder: Ernst, Franz. T., Dr, med.
Haubachstrasse 6
D-4600 Dortmund 50(DE)

(72) Erfinder: ten Hompel, Michael, Dipl.-Ing.
Am Sturmwald 40
D-4600 Dortmund 50(DE)

(74) Vertreter: Patentanwälte Meinke und Dabringhaus
Dipl.-Ing. J. Meinke Dipl.-Ing. W. Dabringhaus
Westenhellweg 67
D-4600 Dortmund 1(DE)

(54) Verfahren und Einrichtung zur unblutigen Messung von Blutdruck und Puls, insbesondere beim Menschen.

(57) Mit einem Verfahren und einer Einrichtung zur unblutigen Messung von Blutdruck und Puls, insbesondere beim Menschen soll eine Lösung unter Vermeidung der beschriebenen Nachteile geschaffen werden, mit der sowohl der Blutdruck als auch der Puls in kürzester Zeit auch von ungeschultem Personal, insbesondere auch vom Patienten selber durchgeführt werden können. Dies wird dadurch erreicht, daß die Messung durch Erfassung der Arterienbewegung von außerhalb des Körpers aus erfolgt.

Fig. 1

Croydon Printing Company Ltd.

"Verfahren und Einrichtung zur unblutigen Messung von Blutdruck und Puls, insbesondere beim Menschen"

Die Erfindung richtet sich auf ein Verfahren zur unblutigen Messung von Blutdruck und Puls, insbesondere beim Menschen. Beim Menschen wird üblicherweise der Blutdruck mittels einer Manschette gemessen, die in der Armbeuge eines Patienten um den Arm gelegt wird und anschließend solange aufgepumpt wird, bis der Fluß des Blutes durch die Arterien unterbrochen wird. Der dafür erforderliche Druck wird mittels eines Manometers ermittelt. Anschließend wird der Druck langsam ermäßigt, bis das Blut wieder ungedrosselt fließen kann. Auch dieser Druck wird mittels des Manometers gemessen. Anhand der Fließgeräusche des Blutes durch die Arterie werden vom Arzt mittels eines Stethoskopes die der Druckmessung zugrundeliegenden Zustände der Arterie ermittelt.

Die Pulsfrequenz wird durch einfaches Auszählen der einzelnen Pulsschläge während eines vorgehenden Zeitintervalls bestimmt. Die Messungen nach dem bekannten Verfahren können nur von geschultem Personal durchgeführt werden und beanspruchen einen nicht unerheblichen Teil der vom Arzt für die Betreuung eines Patienten eingeplanten Zeit. Die erforderlichen Gerätschaften sowie das Verfahren selbst rufen insbesondere bei Kindern Mißtrauen hervor, das durch zusätzlichen Zeitaufwand zunächst vom Arzt zerstreut werden muß.

Aufgabe der Erfindung ist die Schaffung einer Lösung unter Vermeidung der beschriebenen Nachteile ein Verfahren und eine Einrichtung zu schaffen, mit der sowohl der Blutdruck als auch der Puls in kürzester Zeit auch von ungeschultem Personal, insbesondere auch vom Patienten selber durchgeführt werden können. Eine von vielen Patienten als besonders unangenehm empfundene Druckaufbringung über die Manschette soll möglichst vermieden und daher das Verfahren unter anderem auch für Kinder besonders geeignet sein, wobei die Blutdruckmeßeinrichtung auch den Einsatz von Peripheriegeräten möglich machen soll.

Diese Aufgabe wird gemäß der Erfindung bei einem Verfahren der eingangs bezeichneten Art dadurch gelöst, daß die Messung durch Erfassung der Arterienbewegung von außerhalb des Körpers aus erfolgt.

Der Vorteil der Erfindung besteht darin, daß die zu erfassenden Größen im wesentlichen auf die Arterienbewegung reduziert werden, so daß eine entsprechende Messung in wesentlich kürzerer Zeit und wesentlich vereinfacht durchgeführt werden kann. Die Erfassung der Arterienbewegung kann auch durch ungeübtes Personal erfolgen. Ein weiterer entscheidender Vorteil liegt aber darin, daß sie über längere Zeiten ohne Störung durchgeführt werden kann und daß die gerade über Langzeitmessungen erfaßbaren Belastungen für den Patienten durch Eingriffsmaßnahmen von außen behoben werden kann.

In Ausgestaltung sieht die Erfindung vor, daß nach Erfassung der Arterienbewegung das so erhaltene Signal in ein Druck- und/oder Wege- und/oder Pulsfrequenzsignal umgerechnet wird. Druck- und/oder Wegesignale lassen sich mit bekannter Technik in einfachen elektrischen Größen darstellen. Diese Größen sind dann mit herkömmmlicher Technik weiter verarbeitbar, insbesondere mit Kleincomputern, die in der Regel heute in jeder Arztpraxis zur Verfügung stehen oder selbst bei einer Vielzahl von potentiellen Patienten bereits verfügbar sind.

Die Erfindung sieht auch vor, daß ein Sensor unmittelbar über eine mechanische oder fluidische Kopplung von der Arterienbewegung beaufschlagt wird. Über derartige Kopplungen wird erreicht, daß mögliche Fehlerquellen, z.B. durch die Armbewegung der jeweiligen Person, verringert oder ausgeschlossen werden, so daß nur die wirkliche Arterienbewegung als zugrundeliegende Meßgröße eingesetzt werden kann, wobei je nach Rechenmodell auch zusätzliche Informationen für die Weiterverarbeitung des Signales einsetzbar sind. Derartige Größen können z.B. sein der real herrschende Umgebungsdruck an der Meßstelle, die Körpertemperatur

- 4 -

0240735

des Patienten, ggf. der Augendruck u. dgl. mehr.

In besonderer Ausgestaltung sieht die Erfindung vor, daß der Sensor über einen vorbestimmbaren Offset-Druck die Arterie beaufschlagt und die Messung des Offset-Druckes sowie der im Sensor erfolgten Impulse zur Berechnung des Blutdruckes verwendet wird.

Es hat sich gezeigt, daß das Aufbringen eines Vordruckes (Offset-Druck) auf das Meßgerät relativ z.B. zum Unterarm des Patienten die Messung stark vereinfacht, da dieser Wert von außen vorbestimmbar ist und der Sensor dann relativ zu seinem bekannten Anpreßdruck die Arterienbewegung weitermeldet und damit ein Signal erzeugt wird, welches zur Bestimmung des Blutdruckes direkt herangezogen werden kann.

Eine andere Art der Kompensation besteht darin, daß in einem Meßkreis wenigstens zwei Sensoren vorgesehen werden, wobei ein Sensor die zu messende Arterie und wenigstens einer der weiteren Sensoren einen pulsneutralen Bereich der Körperoberfläche beaufschlagt, wobei auch in diesem Falle die Sensoren mit vorbestimmten Anpreßdrücken an die Körperoberfläche der zu messenden Person anpreßbar sind.

Die Erfindung sieht auch vor, daß über einen Rechner eine Extremwertbildung und/oder eine Fourieanalyse des Verlaufs der Arterienbewegung mit ggf. einer Mittelwertbildung, einer gleichzeitigen Ermittlung der Pulsfrequenz, einer Korrekturwertbildung, einer Ermittlung eines mittleren Korrekturwertes, mit einer Speicherung der ermittelten Werte und/oder mit einem Ausdruck der ermittelten Werte in digitaler oder analoger Darstellung über einen Drucker erfolgt.

Diese Verfahrensweisen stellen die Bearbeitungsweisen einer Rechenanlage für den hier vorgesehenen erfinderischen Zweck dar. Auswertungen dieser erfinderischen Vorgehensweisen sind weiter unten anhand von Computerzeichnungen näher erläutert.

Weitere Vorteile bestehen darin, daß sich aus den Extremwerten die Blutdruckwerte des Patienten ermitteln lassen, ohne daß es der Erfassung der Fließgeräusche bedürfte. Auch läßt sich über eine Mittelwertbildung der Einfluß unterschiedlicher Fehlerquellen ausschließen. Die synchrone Erfassung der Pulsfrequenz läßt ebenfalls nicht nur eine Mittelwertbildung zu, sondern ermöglicht auch die Kompensierung von Meßfehlern über eine gewisse Zeit, die sich nach der Pulsfrequenz richtet, wobei die Speicherung und die ggf. optische Wiedergabe durch Ausdruck der Meßwerte für den Diagnostiker das notwendige Informationsmaterial bereitstellt.

In einer für die Erfindung wesentlichen Ausgestaltung ist vorgesehen, daß eine Blutdruck-/Pulsmessung kontinuierlich erfolgt und/oder daß die ermittelten Meßwerte zur Steuerung und/oder zur Regelung einer Medikamentenpumpe eingesetzt werden.

Wie weiter oben bereis angegeben, liegt ein besonderer Vorteil der Erfindung darin, daß über längere Zeiträume kontinuierlich Blutdruck und Pulsfrequenz und auch Art des Druckes oder Pulses gemessen werden können. Dies macht es möglich, dem Patienten direkt eine Medikamentenpumpe zuzuordnen, die bei auftretenden Störungen den Patienten mit den notwendigen Medikamenten versorgt. Damit ist es beispielsweise möglich, eine entsprechende Einrichtung etwa in Art einer Armbanduhr am Handgelenk zu tragen, wobei die Blutdruck- und Pulserfassungseinrichtung der entsprechenden Arterie zugeordnet ist und im gleichen Gerät eine kleine Pumpe zum Einspritzen von Medikamenten eingebaut ist.

Die Erfindung sieht daher auch eine Vorrichtung zur Durchführung des Verfahrens vor, welche sich dadurch auszeichnet, daß der Druckaufnehmer als elektronisches Bauteil ausgebildet ist und eine elastische Lagerung in einem Gehäuse aufweist. Elektronische Druckaufnehmer stehen schon in unterschiedlichen Bauarten zur Verfügung. Wesentlich ist die spezielle Lagerung dieser Bauteile für den vorliegenden Zweck. Die elastische Lagerung in einem Gehäuse, es kann sich hier z.B. um eine Silikonmasse oder ein ähnliches Material handeln, ermöglicht es, die Messung weitestgehend von Fremdeinflüssen unabhängig zu machen.

In Ausgestaltung sieht daher die Erfindung auch vor, daß der Druckaufnehmer mit einem Verstärker, einem A/D-Wandler und einer Rechenanlage verbunden ist, wobei letztere ggf. ein Anzeigegerät und/oder einen Drucker aufweist. Bei dieser Schaltung werden im wesentlichen herkömmliche Bauteile eingesetzt, wie ein Verstärker und ein Analog-/Digitalwandler u. dgl., es hat sich aber bei Versuchen gezeigt, daß diese Steuerungsaufbauten besonders zweckmäßig sind.

Die Erfindung sieht auch vor, daß der Druckaufnehmer über eine Manschette oder dgl. mit vorbestimmtem bzw. vorbestimmbarem Offset-Druck im Bereich einer Arterie am Körper andrückbar ist. Die damit verbundenen Vorteile wurden weiter oben bereits beschrieben. Der vorbestimmbare Offset-Druck dient dem Rechner zum Ausgleich von Fehlerquellen, so daß gewährleistet ist, daß der sich durch die Arterienbewegung auf den Druckaufnehmer übertragene Impuls auf den Blutdruck direkt umrechenbar ist.

Zweckmäßig kann es sein, wenn der Druckaufnehmer in einem Gehäuse unter vorbestimmbarer Federkraft auf die Hautoberfläche aufdrückbar ist, wobei in weiterer Ausgestaltung vorgesehen sein kann, daß die Federn an einer Seite das elastische Lagermittel des Sensors mit beispielsweise einem Silikonkörper beaufschlagt und mit ihrer anderen Seite fest oder wiederum elastisch am Gehäuse des Druckaufnehmers angeordnet ist. Diese Gestaltungen dienen insbesondere zur Fehlerkompensation.

Wie weiter oben bereits angegeben, eignet sich eine derartige Vorrichtung insbesondere zur Steuerung einer Medikamentenpumpe, so daß die Erfindung auch vorsieht, daß die Recheneinheit eine Einrichtung zur Steuerung und/oder Regelung einer derartigen Medikamentenpumpe aufweist.

Die Erfindung ist nachstehend anhand der Zeichnung beispielsweise näher erläutert, diese zeigt in

Fig. 1 die Lage eines Druckaufnehmers über der Arteria radialis,

Fig. 2 eine vergleichbare Anordnung mit einem Differenzdruckaufnehmer,

Fig. 3 ein Ausführungsbeispiel eines Druckaufnehmers,

Fig. 4 ein weiteres Ausführungsbeispiel eines Druckaufnehmers,

Fig. 5   einen Druckaufnehmer im Schnitt,

Fig. 6   einen Druckaufnehmer in Aufsicht,

Fig. 7   ein Blockschaltbild der Meßeinrichtung sowie in

Fig. 8   die Druck-/Pulsverlaufskurve eines Patienten in
         Ruhe,

Fig. 9   die gleiche Kurve eines Patienten nach einer Kurzbelastung,

Fig. 10  den Druck-/Pulsverlauf einer Person vor Anlegen
         einer Oberarmmanschette bei langsamen Aufbringen
         eines entsprechenden Druckes in die Manschette sowie in

Fig. 11  eine Vergrößerung des rechten Teiles der Kurve gemäß Fig. 10.

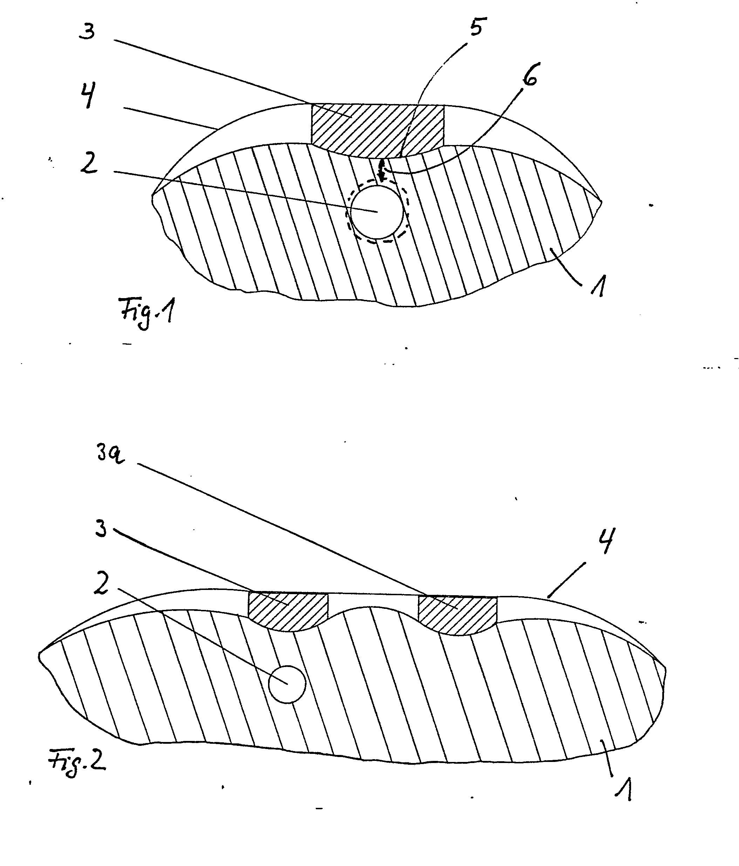

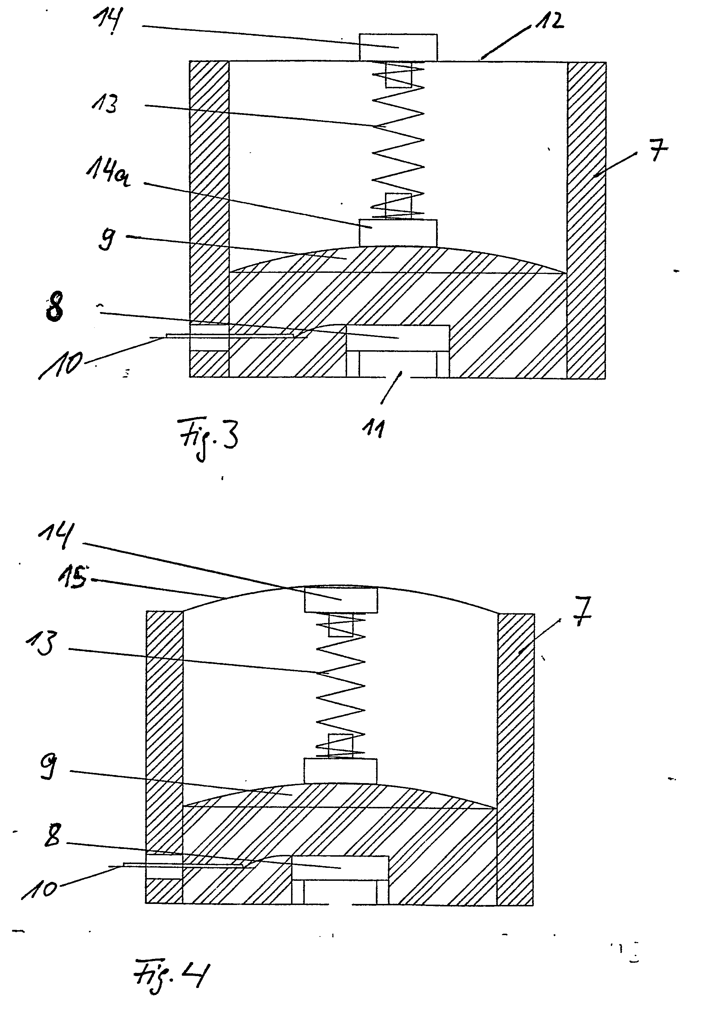

Auf einen in Fig. 1 und 2 nur andeutungsweise wiedergegebenen menschlichen Arm 1 mit einer Arterie 2 ist ein Druckaufnehmer 3 aufgesetzt und dort mit einer andeutungsweise
wiedergegebenen Manschette 4 unter Aufbringung eines Vor-
(Offset)-Druckes festgelegt.

Erkennbar wird durch eine den Blutdruckschwankungen entsprechende Formänderung der Arterie 2 (in Fig. 1 gestrichelt dargestellt) ein wechselnder Impuls auf die Unterseite 5 des Druckaufnehmers 3 ausgeübt, was durch den Doppelpfeil angedeutet ist. Diese sich ändernden Bedingung werden vom Druckaufnehmer 3 registriert, in elektrische Signale umgewandelt, über einen Verstärker 7 einem A/D (analog/digital)-Wandler gewandelt und einem Computer 9 zugeführt, dessen Ergebnisse ggf. über einen dort angeschlossenen Drucker 10 ausgedruckt werden können.

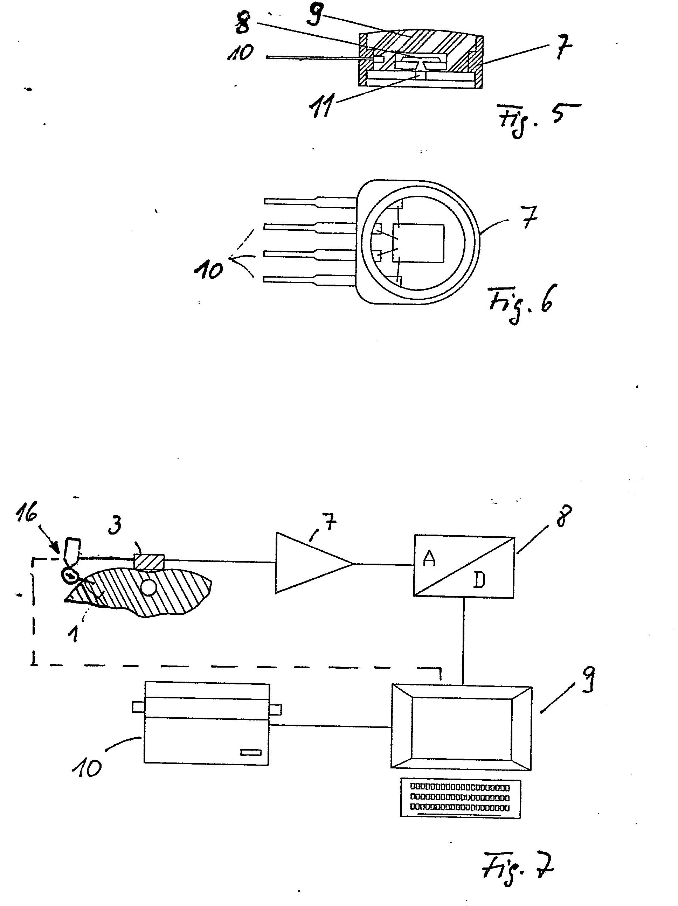

Der nähere Aufbau von Druckaufnehmern 3 ist in Verbindung mit den Fig. 3 - 6 weiter unten näher beschrieben.

In Fig. 2 ist eine abgewandelte Art wiedergegeben: Hier sind zwei Druckaufnehmer 3 und 3a vorgesehen, wobei einer der Druckaufnehmer 3, in der Nähe der Arterie 2 angeordnet ist, während der Druckaufnehmer 3a in einem von deren Pulsieren weitestgehend ungestörten Nachbarbereich angeordnet ist. Beide Druckaufnehmer sind wieder mittels einer Manschette 4 um den Unterarm einer Person gelegt, der auch hier mit 1 bezeichnet ist. Mit einer entsprechenden Schaltung können die differierenden Signale aus dem Druckaufnehmer 3 und dem Druckaufnehmer 3a zur Kompensation der jeweiligen Meßwerte herangezogen werden, um so eine möglichst hohe Meßgenauigkeit zu erreichen.

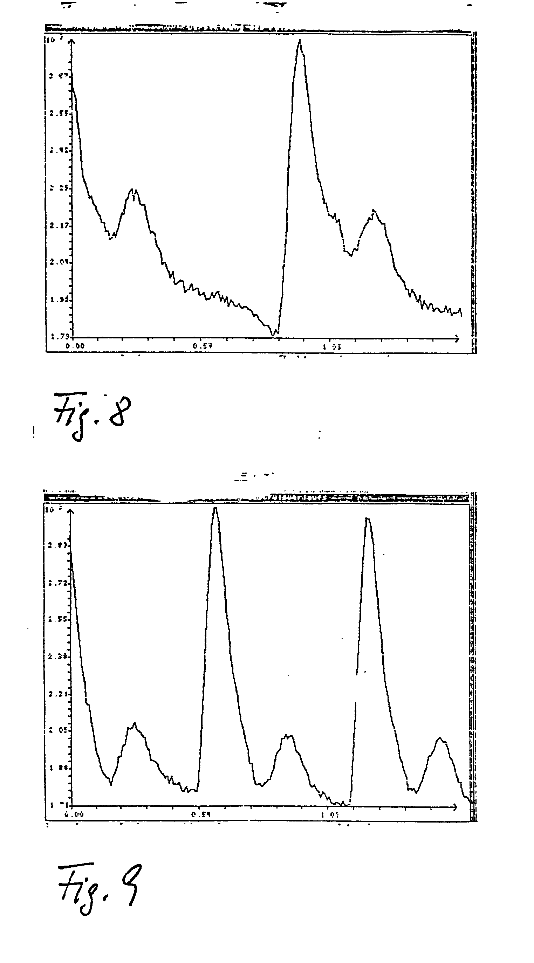

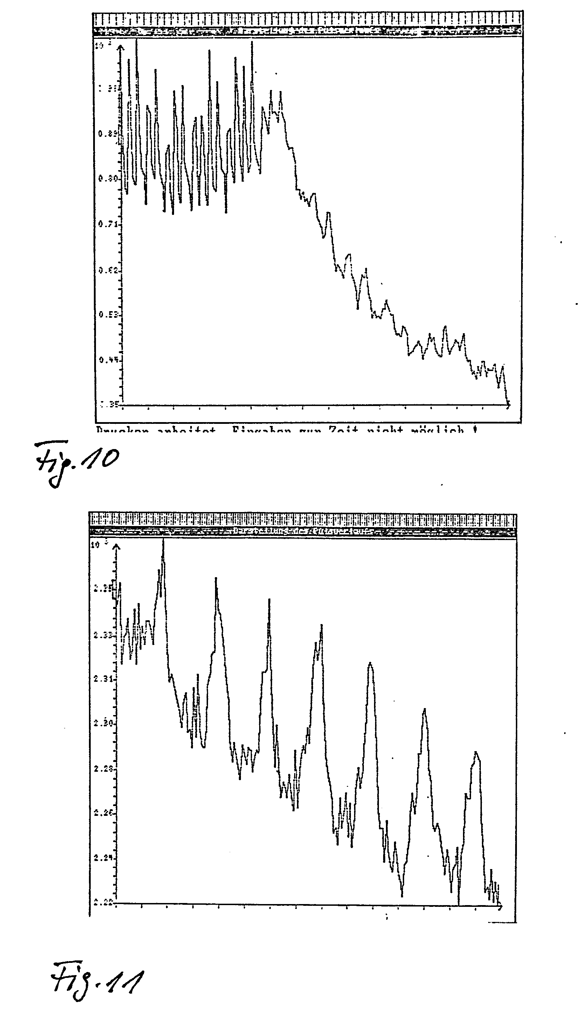

In den Fig. 5 und 6 ist der im Druckaufnehmer 3 eingebrachte Drucksensor etwas näher beschrieben. In einem Gehäuse 7 ist ein elektronischer Sensor 8 in einer Silikonmasse 9 untergebracht. Die elektrischen Anschlüsse sind mit 10 bezeichnet. Eine Öffnung 11 dient zum Ausgleich des atmosphärischen Druckes (Fig. 5).

Die Art des Einbaus eines derartigen Sensors 8 ist in den Fig. 3 und 4 in unterschiedlichen Ausführungen wiedergeben. Dort ist innerhalb eines von einer festen Abdeckung 12 abgeschlossenen Gehäuses 7 der Sensor 8 im Silikonbett 9 eingegossen. Die Besonderheit besteht hierbei in einer Federlagerung über eine Feder 13, die zwischen zwei Federkolben 14 und 14a eingespannt ist. Damit läßt sich beispielsweise ein vorbestimmbarer Offset-Druck im Gehäuse 7 simulieren, um Meßfehler ausgleichen zu können.

In Fig. 4 ist ein abgewandeltes Ausführungsbeispiel dargestellt, wobei alle gleichen Bauteile das gleiche Bezugszeichen tragen. Der wesentliche Unterschied besteht hier in der Gestaltung der Abdeckung des Gehäuses 7 . Diese Abdeckung besteht aus einer elastischen Membran 15, die hier wiederum zur weiteren Fehlkompensation dient.

In Fig. 8 ist der Druckverlauf eines Patienten in Ruhelage dargestellt, wobei in Fig. 9 der Pulsverlauf des gleichen Patienten nach Kurzbelastung wiedergegeben ist. Hier ist zu beachten, daß der Amplitudenmaßstab rechnerisch in etwa gleich gemacht wurde, während er in Wirklichkeit deutlich differiert. Die Kurven zeigen nicht nur die typische Druckkurve, sondern offenbaren dem Diagnostiker auch weitere Einzelheiten durch die spezielle Kurvengestaltung.

Dies gilt z.B. auch für die beiden Fig. 10 und 11, in denen das Abdrücken der Arterie am Oberarm dargestellt ist, gemessen durch einen Druckaufnehmer an der Arteria radialis. Hier wird nicht nur der deutliche Druckabfall erkennbar, sondern bei der Vergrößerung der abfallenden Kurve gemäß Fig. 10 in Fig. 11 auch Einzelheiten des Pulsverhaltens.

Wesentlich für die Erfindung ist auch, daß mit dem Druckaufnehmer und der elektronischen Überwachung der Werte eine Medikamentenpumpe 16 (Fig. 7) möglich gemacht wird. Damit ist es möglich, einem kranken Patienten z.B. bei Überschreiten von gespeicherten Sollwerten ein dämpfendes oder stimulierendes Medikament direkt von außen zuzuführen.

Ansprüche:

1. Verfahren zur unblutigen Messung von Blutdruck und Puls, insbesondere beim Menschen, dadurch gekennzeichnet, daß die Messung durch Erfassung der Arterienbewegung von außerhalb des Körpers aus erfolgt.

2. Verfahren nach Anspruch 1, dadurch gekennzeichnet, daß nach Erfassung der Arterienbewegung das so erhaltene Signal in ein Druck- und/oder Wege- und/oder Pulsfrequenzsignal umgerechnet wird.

3. Verfahren nach Anspruch 1 oder 2, dadurch gekennzeichnet, daß ein Sensor mittelbar über eine mechanische oder fluidische Kopplung von der Arterienbewegung beaufschlagt wird.

4. Verfahren nach einem der vorangehenden Ansprüche, dadurch gekennzeichnet, daß der Sensor über einen vorbestimmbaren Offsetdruck die Arterie beaufschlagt und die Messung des Offsetdruckes sowie der im Sensor erfolgten Impulse zur Berechnung des Blutdruckes verwendet wird.

5. Verfahren nach einem der vorangehenden Ansprüche,

dadurch gekennzeichnet,

daß in einem Meßkreis wenigstens zwei Sensoren vorgesehen werden, wobei ein Sensor die zu messende Arterie und wenigstens einer der weiteren Sensoren einen pulsneutralen Bereich der Körperoberfläche beaufschlagt.

6. Verfahren nach einem der vorangehenden Ansprüche,

dadurch gekennzeichnet,

daß über einen Rechner eine Extremwertbildung und/oder eine Fourieanalyse des Verlaufs der Arterienbewegung mit ggf. einer Mittelwertbildung, einer gleichzeitigen Ermittlung der Pulsfrequenz, einer Korrekturwertbildung, einer Ermittlung eines mittleren Korrekturwertes, mit einer Speicherung der ermittelten Werte und/oder mit einem Ausdruck der ermittelten Werte in digitaler oder analoger Darstellung über einen Drucker erfolgt.

7. Verfahren nach einem vorangehenden Ansprüche,

dadurch gekennzeichnet,

daß eine Blutdruck-/Pulsmessung kontinuierlich erfolgt und/oder daß die ermittelten Meßwerte zur Steuerung und/ oder zur Regelung einer Medikamentenpumpe eingesetzt werden.

8. Vorrichtung zur Durchführung des Verfahrens nach einem der vorangehenden Ansprüche,

dadurch gekennzeichnet,

daß der Druckaufnehmer (3) als elektronisches Bauteil (8) ausgebildet ist und eine elastische Lagerung (9) in einem Gehäuse (7) aufweist.

9. Vorrichtung nach Anspruch 8,

dadurch gekennzeichnet,

daß der Druckaufnehmer (3) mit einem Verstärker (7), einem A/D- Wandler (8) und einer Rechenanlage (9) verbunden ist, wobei letztere ggf. ein Anzeigegerät und/oder einen Drukker (10) aufweist.

10. Vorrichtung nach Anspruch 8 oder 9,

dadurch gekennzeichnet,

daß der Druckaufnehmer (3) über eine Manschette (4) o. dgl. mit vorbestimmten bzw. vorbestimmbaren Offsetdruck im Bereich einer Arterie (2) am Körper anbringbar ist.

11. Vorrichtung nach Anspruch 8 oder einem der folgenden, dadurch gekennzeichnet,

daß der Druckaufnehmer (3 bzw. 8) in einem Gehäuse (7) unter vorbestimmbarer Federkraft auf die Hautoberfläche aufdrückbar ist.

12. Vorrichtung nach Anspruch 11,

dadurch gekennzeichnet,

daß die Feder (13) an einer Seite das elastische Lagermittel (9) des Sensors (8) mit beispielsweise einem Silikonkörper beaufschlagt und mit ihrer anderen Seite fest (12)
oder wiederum elastisch (15) am Gehäuse (7) des Druckaufnehmers (3) angeordnet ist.

13. Vorrichtung nach Anspruch 8 oder einem der folgenden,
dadurch gekennzeichnet,
daß die Recheneinheit (9) eine Einrichtung zur Steuerung
und/ oder Regelung einer Medikamentenpumpe (16) aufweist.

# PATENTANWÄLTE MEINKE UND DABRINGHAUS

ZUGELASSEN BEIM EUROPÄISCHEN PATENTAMT · EUROPEAN PATENT ATTORNEYS · MANDATAIRES EN BREVETS EUROPÉENS

An das

Europäische Patentamt

Erhardtstraße 27

8000 München 2

DIPL.-ING. J. MEINKE
DIPL.-ING. W. DABRINGHAUS

4600 DORTMUND 1,  20. März 87
WESTENHELLWEG 67

D/S

TELEFON       (0231) 14 50 71
TELEGRAMM DOPAT Dortmund
TELEX       . 822 7328 pat d
TELEFAX     (0231) 14 76 70

AKTEN-NR.:   1E/5838

87 103 210.8
Dr. F. T. Ernst und Prof. R. Jünemann

In obiger Angelegenheit werden vorschriftsmäßige Zeichnungen übermittelt mit der Bemerkung, daß sich in die Beschreibung, insbesondere in bezug auf Fig. 7, ein Bezeichnungsfehler eingeschlichen hat. Die Bezugszeichen in Fig. 7 "7, 8, 9 u. 10" müssen abgeändert werden in die Bezugszeichen "17, 18, 19 u. 20". Dies gilt dann entsprechend für Anspruch 9 und dessen Bezugszeichen sowie den ersten Absatz der Beschreibung Seite 10 des eingereichten Textes (Kopie anbei).

Wir bitten, das vorgekommene Versehen zu entschuldigen und die Korrektur amtsseitig zu berücksichtigen.

Patentanwalt Dabringhaus

Anlage
Zeichnungen (3-fach),
neue Seiten 10 und 15 (3-fach)

Fig.1

Fig.2

Fig. 3

Fig. 4

8

9

10

7

11

*Fig. 5*

7

10

*Fig. 6*

16

3

7

8

A
D

1

9

10

*Fig. 7*

Fig. 8

Fig. 9

Fig. 10

Fig. 11